# FASCICULE DE BREVET EUROPEEN

(11) **EP 0 966 426 B1**
(45) Date de publication et mention de la délivrance du brevet: **26.09.2001**
(21) Numéro de dépôt: 98942808.1
(22) Date de dépôt: 01.09.1998
(51) Int. Cl.: C07C 229/16, C07C 235/06, A61K 31/195, A61K 7/48

(54) **NOUVEAUX COMPOSES DERIVES D'ACIDE ALKYLENE DIAMINE DI- OU TRI-ACETIQUE, LEUR PROCEDE DE PREPARATION, LEUR UTILISATION DANS DES COMPOSITIONS COSMETIQUES ET PHARMACEUTIQUES, ET COMPOSITIONS LES COMPRENANT**
ALKYKENDIAMIN DI- ODER TRI-ESSIGSÄUREDERIVATE, VERFAHREN ZU IHRER HERSTELLUNG UND IHRE VERWENDUNG IN KOSMETISCHEN ZUSAMMENSETZUNGEN
NOVEL DIAMINE ALKYLENE DIACETIC OR TRIACETIC ACID DERIVATIVES, PREPARATION METHOD, USE IN COSMETIC AND PHARMACEUTICAL COMPOSITIONS AND COMPOSITIONS CONTAINING THEM

(30) Priorité: 09.09.1997 FR 9711174
(43) Date de publication de la demande: 29.12.1999
(73) Titulaire: L'OREAL, 75008 Paris (FR)
(72) Inventeur: GALEY, Jean-Baptiste, F-93600 Aulnay (FR); DUMATS, Jacqueline, F-93420 Villepinte (FR)
(74) Mandataire: Dodin, Catherine
(86) Numéro de dépôt international: FR9801877
(87) Numéro de publication internationale: WO9912891

(56) Documents cités:
- EP-A- 0 700 896
- EP-A- 0 755 917
- WO-A-94/11338
- WO-A-95/16663
- US-A- 4 528 196
- GALEY, JEAN-BAPTISTE ET AL: "N,N'-bis(3,4,5-trimethoxybenzyl) ethylenediamine N,N'-diacetic acid as a new iron chelator with potential medicinal applications against oxidative stress" BIOCHEM. PHARMACOL. (1996), 51(2), 103-15 CODEN: BCPCA6;ISSN: 0006-2952,1996, XP002064715

## Description

La présente invention a pour objet de nouveaux composés de type esters dérivés d'acide alkylène diamine di- ou tri-acétique, et notamment leur utilisation dans des compositions cosmétiques et pharmaceutiques en particulier pour protéger l'organisme contre le stress oxydant.
Le stress oxydant caractérise un certain nombre de situations physiologiques et physiopathologiques au cours desquelles existe un déséquilibre de la balance antioxydant/prooxydant. Ce déséquilibre se traduit essentiellement par des processus oxydatifs non contrôlés au sein des tissus vivants mettant en jeu des radicaux libres oxygénés et conduisant à la formation de dégâts oxydatifs sur les molécules et macromolécules biologiques.
Un certain nombre de situations physiopathologiques provoquent, favorisent, accompagnent et/ou sont la conséquence directe d'un stress oxydant. Il s'agit notamment de l'inflammation, du vieillissement, des maladies neurodégénératives. de l'exposition aux UV et aux rayonnements ionisants, de la carcinogenèse, de la toxicité et/ou du mode d'action de certains médicaments.
On sait qu'au cours d'un stress oxydant, du fer est libéré de ses sites de stockage normaux comme la ferritine et devient alors accessible pour participer à certaines réactions, et notamment aux réactions de Fenton et Haber-Weiss, permettant ainsi la formation de radicaux hydroxyles, ceux-ci étant connus pour être responsables de nombreux dommages oxydatifs.
On a, depuis longtemps, recherché des composés, notamment de synthèse, permettant de protéger l'organisme contre le stress oxydant.
Ces composés peuvent être regroupés dans les principales classes suivantes :
- les antilipoperoxydants, tels que la vitamine E, le trolox, le butylhydroxytoluène
- les réducteurs biologiques, comme le glutathion réduit et ses dérivés, la vitamine C et ses dérivés,
- les désactivateurs d'oxygène singulet (quenchers), comme le β-carotène,
- les systèmes capables de décomposer le peroxyde d'hydrogène et, en particulier, des enzymes telles la catalase ou des peroxydases en présence de leurs cosubstrats,
- les systèmes de protection contre l'anion superoxyde, comme la superoxyde dismutase (SOD) ou des SOD-like tels que le complexe Mn-desferal ou le diisopropyl salicylate de cuivre,
- les systèmes capables de décomposer les hydroperoxydes organiques comme la glutathion peroxydase ou des systèmes modèles à base de sélénium,
- les chelateurs du fer comme le desferal ou certaines hydroxypyridinones.
Toutefois, il s'est avéré qu'aucun des chelateurs du fer connus n'était réellement satisfaisant en vue de protéger l'organisme vis-à-vis des radicaux hydroxyles. La plupart de ces composés protecteurs présentent une toxicité du fait des interférences avec le métabolisme normal du fer, ce qui en limite l'utilisation.

La demande de brevet WO-94/11338 décrit de nouveaux chelateurs du fer efficaces contre le stress oxydant. Ces composés, susceptibles de former des complexes avec le fer, ont des constantes de stabilité faibles, ce qui diminue par conséquent les risques de toxicité associés à leur utilisation.
L'action de ces composés repose sur un nouveau concept, celui des chelateurs du fer activables en situation de stress oxydant.
L'intérêt de ces composés est notamment de limiter les effets secondaires potentiels. En effet, en situation normale, ces composés possèdent une affinité pour le fer suffisamment faible pour ne pas déplacer le fer des protéines de transport comme la transferrine, à l'inverse de certains autres chelateurs puissants tels que le desféral ou l'HBED qui ont des constantes de stabilité élevées (supérieures à 10³⁰). En situation de stress oxydant, ces composés sont oxydés de manière spécifique par H₂O₂, en espèces possédant une forte affinité pour le fer et empêchant sa participation à la formation de HO°, d'où le terme "d'activation en situation de stress oxydant".
Un certain nombre d'expériences *in vitro* ont confirmé l'intérêt de ces composés en tant que protecteurs vis à vis de l'induction de dommages oxydatifs catalysés par le fer sur les différentes classes de molécules biologiques.
Toutefois, l'effet protecteur de ces composés de l'art antérieur sur des cellules en culture reste relativement modéré car leur biodisponibilité reste faible même lorsque les composés se présentent sous forme d'esters d'alkyle.

La présente invention a pour but de résoudre ce problème en proposant de nouveaux composés qui sont efficaces contre le stress oxydant et qui confèrent une protection beaucoup efficace contre la toxicité de H₂O₂ sur des cellules en culture.

L'invention a donc pour objet un composé de formule (I) : 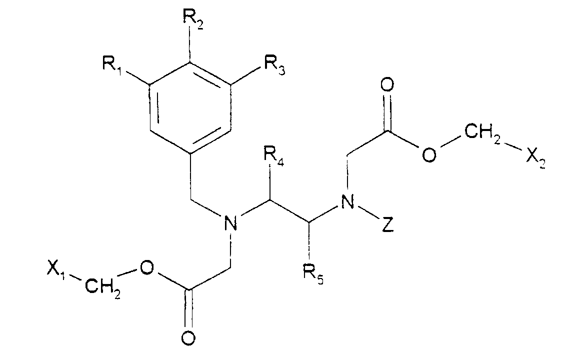 dans laquelle:
- R₁, R₂ et R₃ sont, indépendamment les uns des autres, choisis parmi H. OH ou un radical alcoxy, linéaire ou ramifié, en C₁-C₈
- R₄ et R₅ sont, indépendamment l'un de l'autre, choisis parmi H ou un radical alkyle, linéaire ou ramifié, en C₁-C₄; R₄ et R₅ pris ensemble pouvant former un cycle à 5 ou 6 chaînons
- X₁ et X₂ sont, indépendamment l'un de l'autre, choisis parmi :
   - un groupement -(CO)NR₆R₇ dans lequel R₆ et R₇ sont, indépendamment l'un de l'autre, choisis parmi H ou un radical alkyle, linéaire ou ramifié, en C₁-C₄; R₆ et R₇ pris ensemble pouvant former un cycle à 5 ou 6 chaînons, ou
   - un groupement -O(CO)R₈ dans laquelle R₈ est choisi parmi H ou un radical alkyle, linéaire ou ramifié, en C₁-C₈
   - Z est choisi parmi un groupement de formule (II) ou un groupement de formule (III) 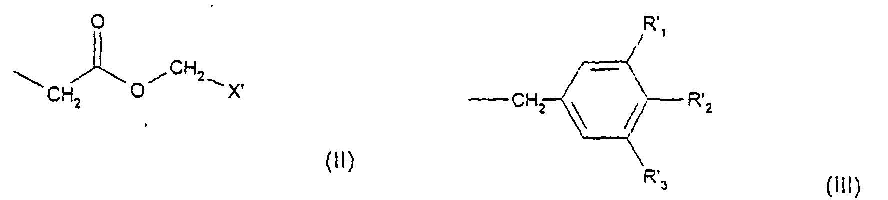 dans lesquels :
      - R'₁, R'₂ et R'₃ sont, indépendamment les uns des autres, choisis parmi H, OH ou un radical alcoxy, linéaire ou ramifié, en C₁-C₈
      - X' est soit un groupement -(CO)NR'₄R'₅ dans lequel R'₄ et R'₅ sont, indépendamment l'un de l'autre, choisis parmi H ou un radical alkyle, linéaire ou ramifié, en C₁-C₄; R'₄ et R'₅ pris ensemble pouvant former un cycle à 5 ou 6 chaînons; soit un groupement -O(CO)R'₆ dans laquelle R'₆ est choisi parmi H ou un radical alkyle, linéaire ou ramifié, en C₁-C₈
ainsi que leurs sels organiques ou minéraux.

L'invention a également pour objet un procédé de préparation des composés ci-dessus dans lequel on fait réagir un sel, par exemple un sel de sodium ou un chlorhydrate, d'un dérivé d'acide N,N'-dibenzyl alkylènediamine N,N'-diacétique ou d'acide N-benzyl alkylenediamine N,N',N'-triacétique, avec 2 à 4 équivalents d'un dérivé halogénométhyle substitué.

L'invention a également pour objet une composition cosmétique ou pharmaceutique, comprenant dans un véhicule cosmétiquement ou pharmaceutiquement acceptable au moins un composé de formule (I) ci-dessus.

Un autre objet de l'invention est l'utilisation des composés de formule (I) en tant qu'agent antioxydant.
Un autre objet de l'invention est l'utilisation des composés de formule (I) dans une composition cosmétique, pour traiter le stress oxydant et/ou pour traiter les effets de l'exposition au soleil et/ou pour prévenir le vieillissement notamment de la peau ou des cheveux.
Un autre objet de l'invention est l'utilisation des composés de formule (I) pour la préparation d'une composition pharmaceutique destinée à traiter le stress oxydant notamment lié à certains états pathologiques, et/ou destinée à traiter des situations pathologiques telles que les cancers, les états inflammatoires, l'ischémie reperfusion, les surcharges en fer, les maladies dégénératives du système nerveux, et/ou destinée à traiter les effets de l'exposition aux rayonnements ionisants ou solaires, et/ou destinée à traiter les effets de l'utilisation de certains médicaments générateurs de radicaux libres, et/ou destinée à prévenir le vieillissement notamment de la peau ou des cheveux.

On a donc constaté que les composés de l'invention exercent un effet protecteur à des concentrations faibles, de l'ordre de quelques micromoles par litre, alors que les composés de l'art antérieur proche, tel que WO 94/11338, n'exercent d'effet qu'à des concentrations bien supérieures, de l'ordre de la millimole par litre.
Les composés selon l'invention sont donc beaucoup plus efficaces que ceux de l'art antérieur vis à vis de la protection de cellules en culture contre la toxicité de H₂O₂.

Les composés selon l'invention sont donc des esters de dérivés d'acide N,N'-dibenzyl alkylènediamine N,N'-diacétique ou d'acide N-benzyl alkylenediamine N,N',N'-triacétique.

Ils répondent à la formule générale (I) ci-dessus.
De préférence, les radicaux alcoxy sont choisis, indépendamment les uns des autres, parmi les radicaux méthoxy, éthoxy, n-propyloxy, isopropyloxy, n-butyloxy. sec-butyloxy ou tert-butyloxy.
Les radicaux alkyles sont de préférence choisis, indépendamment les uns des autres, parmi les radicaux méthyle, éthyle, n-propyle, isopropyle, n-butyle, sec-butyle ou tert-butyle. Lorsqu'ils forment un cycle, ledit cycle est de préférence un cyclohexyle.

Les composés selon l'invention peuvent aussi se présenter sous forme de sels d'acide minéral ou organique.
Parmi les sels minéraux, on peut citer les sulfates, les chlorhydrates, les nitrates, les phosphates ou les bromates.
Parmi les sels organiques, on peut citer les fumarates, les mésylates, les tosylates.

Parmi les composés préférés selon l'invention, on peut notamment citer:
- le bis acétoxyméthyl-[N,N'-bis(3,4,5-triméthoxybenzyl)]éthylènediamine-N,N'-diacétate,
- le bis pivaloyloxyméthyl-[N,N'-bis(3,4,5-triméthoxybenzyl)]éthylènediamine-N,N'-diacétate, et
- le bis N,N-diéthylaminocarbonylméthyl-[N,N'-bis(3,4,5-triméthoxybenzyl)]-éthylènediamine-N,N'-diacétate,
ainsi que leurs sels, notamment leurs chlorhydrates.

Les composés selon l'invention peuvent être préparés par l'homme du métier sur la base de ses connaissances générales, selon les modes de synthèse conventionnels.
Ce procédé de préparation peut notamment consister à faire réagir un sel, par exemple un sel de sodium ou un chlorhydrate, d'un dérivé d'acide N,N'-dibenzyl alkylènediamine N,N'-diacétique ou d'acide N-benzyl alkylenediamine N,N',N'-triacétique, avec 2 à 4 équivalents d'un dérivé halogénométhyle substitué.
Parmi les dérivés halogénométhyle substitués, on peut citer à titre d'exemple l'acétate de bromométhyle ou le pivalate de chlorométhyle.
La réaction peut avoir lieu dans le DMF à une température de 35-60°C, de préférence 40-50°C, pendant 20-30 heures, de préférence 22-26 heures.
On peut ensuite purifier le produit obtenu, par exemple par chromatographie sur colonne de silice.

Les composés selon l'invention peuvent être utilisés comme substances actives pour se protéger des effets néfastes des radicaux libres, c'est à dire contre le stress oxydant et notamment pour traiter des situations pathologiques en médecine humaine ou vétérinaire telles que les cancers, les états inflammatoires, l'ischémie reperfusion, les surcharges en fer, les maladies dégénératives du système nerveux, ou pour traiter les effets de l'exposition aux rayonnements ionisants ou solaires, ou encore pour traiter les effets de l'utilisation de certains médicaments connus pour générer des radicaux libres, et notamment des anticancéreux comme l'adriamycine.
Ces composés peuvent aussi être utilisés dans des situations non pathologiques telles que l'exposition au soleil ou le vieillissement pour protéger notamment la peau ou les cheveux.

Les compositions, notamment cosmétiques ou pharmaceutiques, comprenant un ou plusieurs composés selon l'invention peuvent se présenter sous diverses formes conventionnelles telles que sous forme d'onguent, de crème, de pommade, de gel, de spray, de lotion, d'émulsion ou de dispersion vésiculaire.
Le composé de formule (I) peut être présent en une quantité de 0,001 à 10 % en poids par rapport au poids total de la composition, de préférence à raison de 0,01 à 8% en poids, préférentiellement de 0,1 à 5% en poids.
Lorsque la composition est une composition pharmaceutique, elle peut être administrée sous toutes les formes d'administration usuelles, telles que par voie orale, topique ou parentérale, le support pharmaceutiquement acceptable étant fonction de la forme d'administration choisie.
La forme galénique ainsi que la quantité de composé présent dans la composition peuvent être aisément déterminées par l'homme du métier sur la base de ses connaissances générales.

Dans les compositions selon l'invention, le composé de formule (I) peut être associé à au moins une autre substance active, notamment une substance anti-radicaux libres).
Ces substances peuvent être choisies parmi :
- les antilipoperoxydants, tels que la vitamine E, le trolox, le butylhydroxytoluène,
- les réducteurs biologiques, comme le glutathion réduit et ses dérivés, la vitamine C et ses dérivés,
- les désactivateurs d'oxygène singulet (quenchers), comme le β-carotène,
- les systèmes capables de décomposer le peroxyde d'hydrogène et, en particulier, des enzymes telles la catalase ou des peroxydases en présence de leurs cosubstrats,
- les systèmes de protection contre l'anion superoxyde, comme la superoxyde dismutase (SOD) ou des SOD-like tels que le complexe Mn-desferal ou le diisopropyl salicylate de cuivre,
- les systèmes capables de décomposer les hydroperoxydes organiques comme la glutathion peroxydase ou des systèmes modèles à base de sélénium,
- les chelateurs du fer comme le desferal ou certaines hydroxypyridinones.
Le composé selon l'invention peut également être associé à des anti-inflammatoires, des filtres UV et/ou des promoteurs de pénétration.

Le composé de formule (I) et la substance active peuvent être associés au sein de la même composition ou être appliqués séparément.

Les exemples ci-après sont donnés en vue d'illustrer la préparation de certains composés de formule (I) et certaines de leurs utilisations dans les domaines pharmaceutiques et cosmétiques.

### Exemple de synthèse 1 : bis acétoxymethyl-[N,N'-bis(3,4,5-trimethoxybenzyl)]-ethylenediamine-N,N'-diacetate de formule

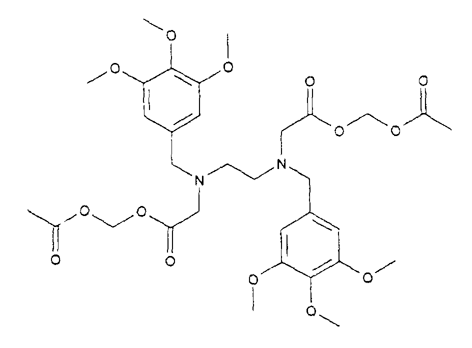

On solubilise 2 g (3,3 mmoles) de dichlorhydrate de l'acide N,N'-bis(3,4,5-trimethoxybenzyl) ethylenediamine-N,N'-diacetique dans 20 ml d'eau par addition de lessive de soude. Le pH est amené à 8 par addition d'acide chlorhydrique concentré. La solution est évaporée à sec, puis le résidu est repris dans 30 ml de dimethyle formamide.
On ajoute 1,3 g de bromométhyle acétate (BrCH₂OCOCH₃; 8,5 mmoles) et le milieu est agité à 45°C pendant 24h. Le mélange est alors évaporé à sec puis repris par du dichlorométhane, lavé à l'eau, séché sur sulfate de sodium, filtré et évaporé à sec.
Le résidu est purifié par chromatographie sur colonne de silice (éluant acétate d'éthyle / heptane 3:1).
On obtient une huile qu'on purifie par cristallisation dans un mélange dichlorométhane / pentane.
On obtient après filtration et séchage, 650 mg d'un solide blanc (rendement : 30%).
Spectre RMN : ¹H et ¹³C (400 MHz) dans CDCl₃ : conforme à la structure attendue

| Analyse élémentaire : | | | | |
|---|---|---|---|---|
| | C | H | N | O |
| % calculé | 56,46 | 6,52 | 4,12 | 32,91 |
| % trouvé | 56,24 | 6,51 | 4,18 | 32,66 |

### Exemple de synthèse 2 : bis pivaloyloxymethyl-[N,N'-bis(3,4,5-trimethoxybenzyl)]ethylenediamine-N,N'-diacetate de formule :

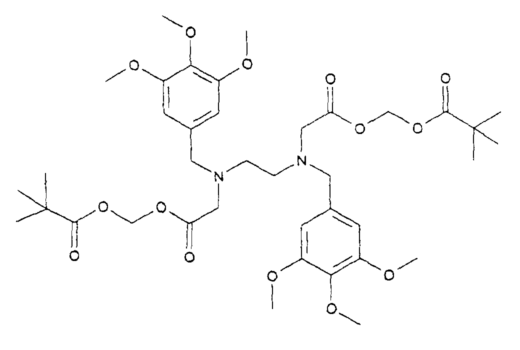

On solubilise 2 g (3,3 mmoles) de dichlorhydrate de l'acide N,N'-bis(3,4,5-trimethoxybenzyl) ethylenediamine-N,N'-diacetique dans 20 ml d'eau par addition de lessive de soude. Le pH est amené à 8 par addition d'acide chlorhydrique concentré. La solution est évaporée à sec, puis le résidu est repris dans 30 ml de dimethyle formamide.
On ajoute 1,5 g de chlorométhyle pivalate (ClCH₂OCOtBu; 10 mmoles) et le milieu est agité à 45°C pendant 24 h. Le mélange est alors évaporé à sec puis repris par du dichlorométhane, lavé à l'eau, séché sur sulfate de sodium, filtré et évaporé à sec. Le résidu est purifié par chromatographie sur colonne de silice (éluant dichlorométhane / méthanol 99:1).
On obtient 500 mg d'une huile incolore (rendement : 20%).
Spectre RMN : ¹H (400 MHz) dans CDCl₃ : conforme à la structure attendue

### Exemple de synthèse 3 : bis N,N-diethylaminocarbonylmethyl-[N,N'-bis(3,4,5-trimethoxybenzyl)]ethylenediamine-N,N'-diacetate dichlorhydrate de formule :

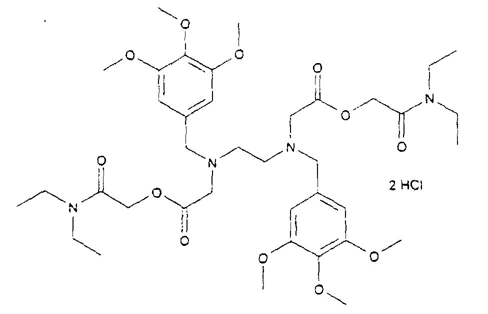

On solubilise 2 g (3,3 mmoles) de dichlorhydrate de l'acide N,N'-bis(3,4,5-trimethoxybenzyl) ethylenediamine-N,N'-diacetique dans 20 ml d'eau par addition de lessive de soude. Le pH est amené à 8 par addition d'acide chlorhydrique concentré. La solution est évaporée à sec, puis le résidu est repris dans 30 ml de dimethyle formamide.
On ajoute 1,0 g de 2-chloro N,N-diethylacétamide (ClCH₂CONEt₂ ; 6.6 mmoles) et 0,1 g d'iodure de méthyle puis le milieu est agité à 45°C pendant 24 h. Le mélange est alors évaporé à sec puis repris par du dichlorométhane, lavé à l'eau, séché sur sulfate de sodium, filtré et évaporé à sec. Le résidu est purifié par chromatographie sur colonne de silice (éluant acétate d'éthyle).
L'huile obtenue est reprise dans l'éthanol. Par addition d'acide chlorhydrique concentré, un précipité blanc apparaît. Celui ci est filtré, lavé à l'éther et séché sous vide.
On obtient 650 mg d'un solide blanc (rendement : 25%).
Spectre RMN : ¹H (400 MHz) dans CDCl₃ : conforme à la structure attendue

### Exemple 4 : mise en évidence de l'activité des composés

L'activité des composés a été évaluée par leur capacité à protéger des cellules V79 contre la toxicité de H₂O₂, technique classiquement utilisée pour évaluer l'efficacité d'antioxydants ou de chelateurs de fer.

On a comparé l'activité du composé de l'exemple 1 avec celle de deux composés de l'art antérieur, décrits dans WO94/11338 :
- comparatif 1 : le dichlorhydrate de l'acide N,N'-bis(3,4,5-trimethoxybenzyl) ethylenediamine-N,N'-diacetique de formule : 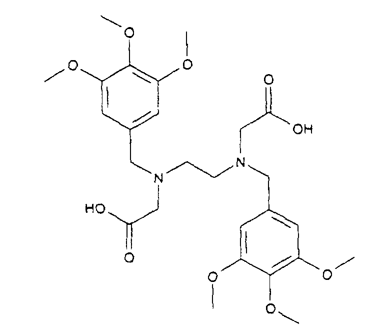
- comparatif 2 : son ester méthylique de formule

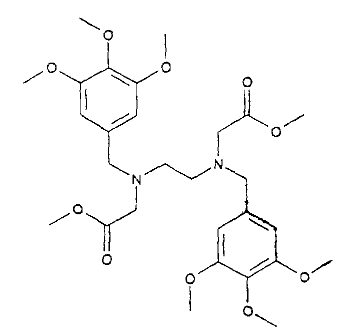

### a/ protocole expérimental

Des cellules V79 ont été repiquées dans le milieu DMEM (Dulbecco Modified Essential Medium) supplémenté par 10% de sérum de veau foetal, streptomycine, pénicilline et glutamine à 37°C sous une atmosphère de 95% air + 5% CO₂.
Les cellules ont ensuite été ensemencées à une densité de 5.10³/ml dans des plaques à 96 puits.
Après 24 heures, les cellules ont été rincées, puis incubées avec les composés à tester à différentes concentrations.
Après 1 heure de contact, 30 µM de H₂O₂ ont été ajoutés et incubés à 37°C pendant encore 1 heure.
Les cellules ont ensuite été rincées, puis remises en contact avec le milieu supplémenté ci-dessus décrit.
Après 72 heures, le nombre de cellules a été évalué dans chaque puits en utilisant la technique du rouge neutre.
L'effet protecteur est évalué par l'IC₅₀, qui mesure la concentration induisant 50% de protection contre la cytotoxicité comparativement aux puits traités par H₂O₂ seul.

### b/ résultats

On a obtenu les résultats suivants :

| Composé | Résultat (concentration induisant 50% de protection) |
|---|---|
| composé de l'exemple 1 | 10 µM |
| comparatif 1 | 25% de protection uniquement à 10 mM |
| comparatif 2 | supérieur à 1 mM (composé insoluble au-delà) |

On constate donc que le composé selon l'invention exerce un effet protecteur beaucoup plus marqué que ceux des deux composés de l'art antérieur.

### Exemple 5

On prépare une composition cosmétique se présentant sous la forme d'une émulsion, en utilisant les constituants ci dessous (% en poids) :

| | |
|---|---|
| composé de l'exemple 1 | 0,1% |
| PEG 50 oxyéthylèné | 3% |
| mono diglyceril stéarate | 3% |
| huile de vaseline | 24% |
| alcool cétylique | 5% |
| eau | qsp 100% |

### Exemple 6

On prépare une composition cosmétique se présentant sous la forme d'une émulsion, en utilisant les constituants ci dessous (% en poids) :

| | |
|---|---|
| composé de l'exemple 1 | 0,1% |
| octyl palmitate | 10% |
| glyceryl isostéarate | 4% |
| huile de vaseline | 24% |
| vitamine E | 1% |
| glycérine | 3% |
| eau | qsp 100% |

### Exemple 7

On prépare la composition cosmétique suivante en utilisant les constituants ci-dessous (% en poids) :

| | |
|---|---|
| composé de l'exemple 2 | 0,05% |
| huile de jojoba | 13% |
| sorbate de potassium | 0,3% |
| cyclopentadimethylsiloxane | 10% |
| alcool stéarylique | 1% |
| acide stéarique | 4% |
| stéarate de polyéthylène glycol | 3% |
| vitamine E | 1% |
| glycérine | 3% |
| conservateurs | qs |
| eau | qsp 100% |

### Exemple 8

On prépare une composition pharmaceutique sous forme de suspension buvable comprenant les constituants suivants :

| | |
|---|---|
| composé de l'exemple 1 | 0,10 g |
| éthanol à 90% | 1,00 g |
| sorbitol à 70% | 0,50 g |
| saccharinate de sodium | 0,01 g |
| p-hydroxybenzoate de méthyle | 0,04 g |
| arôme | qs |
| eau | qsp 5 ml |

### Exemple 9

On prépare une composition pharmaceutique sous forme de comprimé, comprenant les constituants suivants

| | |
|---|---|
| composé de l'exemple 1 | 0,10 g |
| amidon | 0,12 g |
| phosphate bicalcique | 0,20 g |
| lactose | 0,06 g |
| stéarate de magnésium | 0,02 g |

## Revendications

1. Composé de formule (I) : 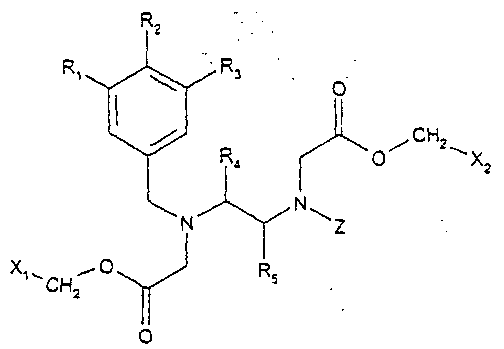 dans laquelle:
- R₁, R₂ et R₃ sont, indépendamment les uns des autres, choisis parmi H, OH ou un radical alcoxy, linéaire ou ramifié, en C₁-C₈
- R₄ et R₅ sont, indépendamment l'un de l'autre, choisis parmi H ou un radical alkyle, linéaire ou ramifié, en C₁-C₄; R₄ et R₅ pris ensemble pouvant former un cycle à 5 ou 6 chaînons
- X₁ et X₂ sont, indépendamment l'un de l'autre, choisis parmi :
- un groupement -(CO)NR₆R₇ dans lequel R₆ et R₇ sont, indépendamment l'un de l'autre, choisis parmi H ou un radical alkyle, linéaire ou ramifié, en C₁-C₄; R₆ et R₇ pris ensemble pouvant former un cycle à 5 ou 6 chaînons, ou
- un groupement -O(CO)R₈ dans laquelle R₈ est choisi parmi H ou un radical alkyle, linéaire ou ramifié, en C₁-C₈
- Z est choisi parmi un groupement de formule (II) ou un groupement de formule (III) 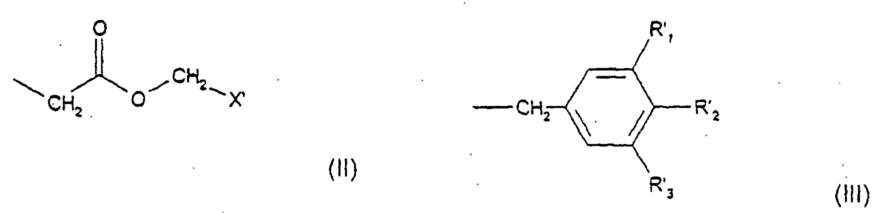 dans lesquels :
- R'₁, R'₂ et R'₃ sont, indépendamment les uns des autres, choisis parmi H, OH ou un radical alcoxy, linéaire ou ramifié, en C₁-C₈
- X' est soit un groupement -(CO)NR'₄R'₅ dans lequel R'₄ et R'₅ sont, indépendamment l'un de l'autre, choisis parmi H ou un radical alkyle, linéaire ou ramifié, en C₁-C₄; R'₄ et R'₅ pris ensemble pouvant former un cycle à 5 ou 6 chaînons; soit un groupement -O(CO)R'₆ dans laquelle R'₆ est choisi parmi H ou un radical alkyle, linéaire ou ramifié, en C₁-C₈
ainsi que leurs sels organiques ou minéraux.

2. Composé selon la revendication 1, dans lequel les radicaux alcoxy sont choisis, indépendamment les uns des autres, parmi les radicaux méthoxy, éthoxy, n-propyloxy, isopropyloxy, n-butyloxy, sec-butyloxy ou tert-butyloxy; et/ou les radicaux alkyles sont choisis, indépendamment les uns des autres, parmi les radicaux méthyle, éthyle, n-propyle, isopropyle, n-butyle, sec-butyle ou tert-butyle.

3. Composé selon l'une des revendications précédentes, dans lequel les sels minéraux ou organiques sont choisis parmi les sulfates, les chlorhydrates, les nitrates, les phosphates, les bromates, les fumarates, les mésylates et les tosylates.

4. Composé selon l'une des revendications précédentes, choisi parmi :
- le bis acétoxyméthyl-[N,N'-bis(3,4,5-triméthoxybenzyl)]éthylènediamine-N,N'-diacétate,
- le bis pivaloyloxyméthyl-[N,N'-bis(3,4,5-triméthoxybenzyl)]éthylènediamine-N,N'-diacétate, et
- le bis N,N-diéthylaminocarbonylméthyl-[N,N'-bis(3,4,5-triméthoxybenzyl)]-éthylènediamine-N,N'-diacétate,
ainsi que leurs sels, notamment leurs chlorhydrates.

5. Procédé de préparation des composés selon l'une des revendications 1 à 4, dans lequel on fait réagir un sel, par exemple un sel de sodium ou un chlorhydrate, d'un dérivé d'acide N,N'-dibenzyl alkylènediamine N,N'-diacétique ou d'acide N-benzyl alkylenediamine N,N',N'-triacétique, avec 2 à 4 équivalents d'un dérivé halogénométhyle substitué.

6. Composition cosmétique ou pharmaceutique, **caractérisée par** le fait qu'elle contient dans un véhicule cosmétiquement ou pharmaceutiquement acceptable au moins un composé de formule (I) selon l'une quelconque des revendications 1 à 4,

7. Composition selon la revendication 6, dans laquelle le composé de formule (I) est présent en une quantité de 0,001 à 10 % en poids par rapport au poids total de la composition, de préférence 0,01 à 8% en poids.

8. Composition selon l'une des revendications 6 à 7, dans laquelle le composé de formule (I) est associé à au moins une autre substance active, notamment une substance anti-radicaux libres.

9. Composition selon la revendication 8, dans laquelle l'autre substance active est choisie parmi les antilipoperoxydants, les réducteurs biologiques, les désactivateurs d'oxygène singulet (quenchers), les systèmes capables de décomposer le peroxyde d'hydrogène, les systèmes de protection contre l'anion superoxyde, les systèmes capables de décomposer les hydroperoxydes organiques, les chelateurs du fer, les anti-inflammatoires, les filtres UV et les promoteurs de pénétration.

10. Composition selon l'une des revendications 6 à 9, se présentant sous forme d'onguent, de crème, de pommade, de gel, de spray, de lotion, d'émulsion ou de dispersion vésiculaire.

11. Utilisation d'au moins un composé de formule (I) selon l'une quelconque des revendications 1 à 4, en tant qu'agent antioxydant.

12. Utilisation d'au moins un composé de formule (I) selon l'une quelconque des revendications 1 à 4, dans une composition cosmétique, pour traiter le stress oxydant et/ou pour traiter les effets de l'exposition au soleil et/ou pour prévenir le vieillissement notamment de la peau ou des cheveux.

13. Utilisation d'au moins un composé de formule (I) selon l'une quelconque des revendications 1 à 4, pour la préparation d'une composition pharmaceutique
- destinée à traiter le stress oxydant notamment lié à certains états pathologiques, et/ou
- destinée à traiter des situations pathologiques telles que les cancers, les états inflammatoires, l'ischémie reperfusion, les surcharges en fer, les maladies dégénératives du système nerveux, et/ou
- destinée à traiter les effets de l'exposition aux rayonnements ionisants ou solaires, et/ou
- destinée à traiter les effets de l'utilisation de certains médicaments générateurs de radicaux libres, et/ou
- destinée à prévenir le vieillissement notamment de la peau ou des cheveux.

## Patentansprüche

1. Verbindung der Formel (I) 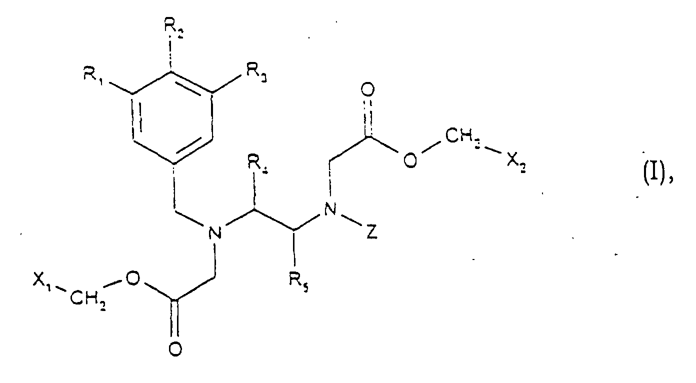 in der bedeuten:
- R₁, R₂ und R₃ unabhängig Reste, die unter Wasserstoff, OH und geradkettigem oder verzweigtem C₁-C₈-Alkoxy ausgewählt sind,
- R₄ und R₅ unabhängig Reste, die unter Wasserstoff und geradkettigem oder verzweigtem C₁-C₄-Alkyl ausgewählt sind, wobei R₄ und R₅ zusammen einen Ring mit 5 oder 6 Ringgliedern bilden können,
- X₁ und X₂ unabhängig Reste, die ausgewählt sind unter:
• -(CO)NR₆R₇, worin R₆ und R₇ unabhängig unter Wasserstoff und geradkettigem oder verzweigtem C₁-C₄-Alkyl ausgewählt sind, wobei R₆ und R₇ zusammen einen Ring mit 5 oder 6 Ringgliedem bilden können, und
• -O(CO)R₈, worin R₈ unter Wasserstoff und geradkettigem C₁-C₈-Alkyl ausgewählt ist,
- Z einen Rest, der unter den Gruppen der Formel (II) und den Gruppen der Formel (III) 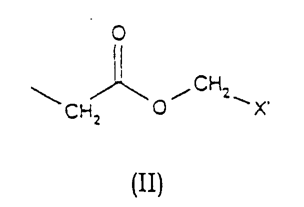 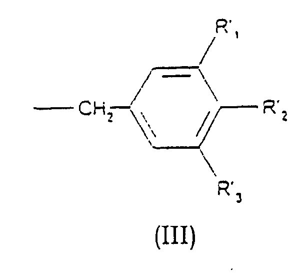 ausgewählt ist, worin bedeuten:
• R'₁, R'₂ und R'₃ unabhängig Reste, die unter Wasserstoff, OH und geradkettigem oder verzweigtem C₁-C₈-Alkoxy ausgewählt sind,
• X' eine Gruppe -(CO)NR'₄R'₅, worin R'₄ und R'₅ unabhängig unter Wasserstoff und geradkettigem oder verzweigtem C₁-C₄-Alkyl ausgewählt sind, wobei R'₄ und R'₅ zusammen einen Ring mit 5 oder 6 Ringgliedern bilden können, oder eine Gruppe -O(CO)R'₆, worin R'₆ unter Wasserstoff und geradkettigem oder verzweigtem C₁-C₈-Alkyl ausgewählt ist
sowie ihre organischen oder anorganischen Salze.

2. Verbindung nach Anspruch 1, wobei die Alkoxyreste unabhängig unter Methoxy, Ethoxy, n-Propyloxy, Isopropyloxy, n-Butyloxy, *sek*.-Butyloxy und *tert*.-Butyloxy und/oder die Alkylreste unabhängig unter Methyl, Ethyl, n-Propyl, Isopropyl, n-Butyl, *sek*.-Butyl und *tert.*-Butyl ausgewählt sind.

3. Verbindung nach einem der vorhergehenden Ansprüche, wobei die anorganischen oder organischen Salze unter den Sulfaten, Hydrochloride, Nitraten, Phosphaten, Bromaten, Fumaraten, Mesylaten und Tosylaten ausgewählt sind.

4. Verbindung nach einem der vorhergehenden Ansprüche, die ausgewählt ist unter
- Bisacetoxymethyl-(N,N'-bis(3,4,5-trimethoxybenzyl)]-ethylendiamin-N,N'-diacetat;
- Bispivaloyloxymethyl-[N,N'-bis(3,4,5-trimethoxybenzyl)]-ethylendiamin-N,N'-diacetat und Bis-N,N-diethylaminocarbonylmethyl-[N,N'-bis(3,4, 5-trimethoxybenzyl)]-ethylendiamin-N,N'-diacetat,
sowie ihren Salzen, insbesonderen ihren Hydrochloriden.

5. Verfahren zur Herstellung der Verbindungen nach einem der Ansprüche 1 bis 4, bei dem ein Salz, beispielsweise ein Natriumsalz oder ein Hydrochlorid, eines Derivats der N,N'-Dibenzylalkylendiamin-N,N'-diessigsäure oder der N-Benzylalkylendiamin-N,N'N'-triessigsäure mit 2 bis 4 Äquivalenten eines substituierten Halogenmethylderivats umgesetzt wird.

6. Kosmetische oder pharmazeutische Zusammensetzung, **dadurch gekennzeichnet, daß** sie in einem kosmetisch oder pharmazeutisch akzeptablen Vehikel mindestens eine Verbindung der Formel (I) nach einem der Ansprüche 1 bis 4 enthält.

7. Zusammensetzung nach Anspruch 6, wobei die Verbindung der Formel (I) in einer Menge von 0,001 bis 10 Gew.-%, bezogen auf das Gesamtgewicht der Zusammensetzung, vorzugsweise 0,01 bis 8 Gew.-%, enthalten ist.

8. Zusammensetzung nach einem der Ansprüche 6 und 7, wobei die Verbindung der Formel (I) mit mindestens einem weiteren Wirkstoff, insbesondere einem Mittel gegen freie Radikale, kombiniert ist.

9. Zusammensetzung nach Anspruch 8, wobei der weitere Wirkstoff unter Antioxidantien für Fette und Öle, biologischen Reduktionsmitteln, Mitteln zur Desaktivierung von Singulett-Sauerstoff (Quencher), Systemen, die imstande sind, Wasserstoffperoxid zu zersetzen, Systemen zum Schutz vor dem Superoxidanion, Systemen, die imstande sind, organische Hydroperoxide zu zersetzen, Chelatbildnern für Eisen, entzündungshemmenden Mitteln, UV-Filtern und Penetrationshilfsmitteln, ausgewählt sind.

10. Zusammensetzung nach einem der Ansprüche 6 bis 9, die in Form einer Salbe, Creme, Pomade, eines Gels, Sprays, einer Lotion, Emulsion oder Vesikeldispersion vorliegt.

11. Verwendung mindestens einer Verbindung der Formel (I) nach einem der Ansprüche 1 bis 4 als Antioxidationsmittel.

12. Verwendung mindestens einer Verbindung der Formel (I) nach einem der Ansprüche 1 bis 4 in einer kosmetischen Zusammensetzung zur Behandlung von oxidativem Stress und/oder zur Behandlung der Auswirkungen der Bestrahlung mit Sonnenlicht und/oder zur Vorbeugng vor der Alterung, insbesondere der Haut oder der Haare.

13. Verwendung mindestens einer Verbindung der Formel (I) nach einem der Ansprüche 1 bis 4 zur Herstellung einer pharmazeutischen Zusammensetzung, die vorgesehen ist für
- die Behandlung von oxidativem Stress, der insbesondere mit bestimmten krankhaften Zuständen verbunden ist, und/oder
- die Behandlung krankhafter Zustände, wie der verschiedenen Krebsarten, der Entzündungen, der Ischämie/Reperfusionsschäden, der zu hohen Eisenkonzentration, der degenerativen Erkrankungen des Nervenssystems, und/oder
- zur Behandlung der Auswirkungen der Bestrahlung mit ionisierender Strahlung oder mit Sonnenlicht und/oder
- zur Behandlung der Auswirkungen der Verwendung bestimmter Arzneimittel, die freie Radikale erzeugen, und/oder
- zur Vorbeugung vor der Alterung, insbesondere Haut oder der Haare.

## Claims

1. Compound of Formula (I): 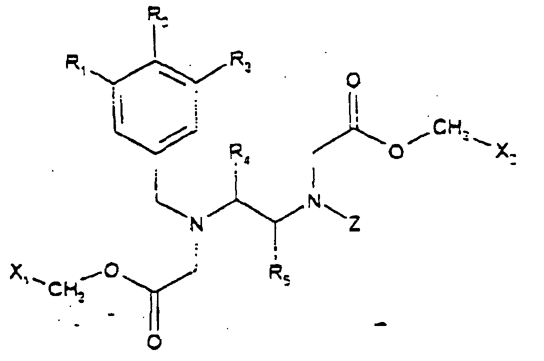 in which:
- R₁, R₂ and R₃ are, independently of each other, chosen from H, OH and a linear or branched C₁-C₈ alkoxy radical
- R₄ and R₅ are, independently of each other, chosen from H and a linear or branched C₁-C₄ alkyl radical; R₄ and R₅, taken together, possibly forming a 5- or 6-membered ring
- X₁ and X₂ are, independently of each other, chosen from
- a group -(CO)NR₆R₇ in which R₆ and R₇ are, independently of each other, chosen from H and a linear or branched C₁-C₄ alkyl radical; R₆ and R₇, taken together, possibly forming a 5- or 6-membered ring, or
- a group -O(CO)R₈ in which R₈ is chosen from H and a linear or branched C₁-C₈ alkyl radical
- Z is chosen from a group of formula A and a group of formula B 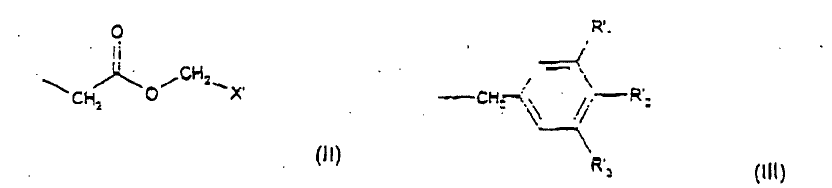 in which:
- R'₁, R'₂ and R'₃ are, independently of each other, chosen from H, OH and a linear or branched C₁-C₈ alkoxy radical
- X' is either a group -(CO)NR'₄R'₅ in which R'₄ and R'₅ are, independently of each other, chosen from H and a linear or branched C₁-C₄ alkyl radical; R'₄ and R'₅, taken together, possibly forming a 5- or 6-membered ring; or a group -O(CO)R'₆ in which R'₆ is chosen from H and a linear or branched C₁-C₈ alkyl radical
and also the organic or mineral salts thereof.

2. Compound according to Claim 1, in which the alkoxy radicals are chosen, independently of each other, from methoxy, ethoxy, n-propyloxy, isopropyloxy, n-butyloxy, sec-butyloxy and tert-butyloxy radicals; and/or the alkyl radicals are chosen, independently of each other, from methyl, ethyl, n-propyl, isopropyl, n-butyl, sec-butyl and tert-butyl radicals.

3. Compound according to one of the preceding claims, in which the mineral or organic salts are chosen from the sulphates, hydrochlorides, nitrates, phosphates, bromates, fumarates, mesylates and tosylates.

4. Compound according to one of the preceding claims, chosen from:
- bis acetoxymethyl-[N,N'-bis(3,4,5-trimethoxybenzyl)]ethylenediamine-N,N'-diacetate,
- bis pivaloyloxymethyl-[N,N'-bis(3,4,5-trimethoxybenzyl)]ethylenediamine-N,N'-diacetate, and
- bis N,N-diethylaminocarbonylmethyl-[N,N'-bis(3,4,5-trimethyoxybenzyl)]ethylenediamine-N,N'-diacetate and also the salts thereof, in particular the hydrochlorides thereof.

5. Process for preparing compounds according to one of Claims 1 to 4, in which a salt, for example a sodium salt or a hydrochloride, of a derivative of N,N'-dibenzylalkylenediamine-N,N'-diacetic acid or of N-benzylalkylenediamine-N,N',N'-triacetic acid is reacted with 2 to 4 equivalents of a substituted halomethyl derivative.

6. Cosmetic or pharmaceutical composition, **characterized in that** it contains, in cosmetically or pharmaceutically acceptable vehicle, at least one compound of Formula (I) according to any one of Claims 1 to 4.

7. Composition according to Claim 6, in which the compound of Formula (I) is present in an amount of from 0.001% to 10% by weight relative to the total weight of the composition and preferably 0.01% to 8% by weight.

8. Composition according to either of Claims 6 and 7, in which the compound of Formula (I) is combined with at least one other active substance, in particular a free-radical-scavenging substance.

9. Composition according to Claim 8, in which the other active substance is chosen from anti-lipoperoxidizing agents, biological reducing agents, singlet-oxygen deactivators (quenchers), systems capable of decomposing hydrogen peroxide, systems for protecting against the superoxide anion, systems capable of decomposing organic hydroperoxides, ionchelating agents, anti-inflammatory agents, UV screening agents and penetration promoters.

10. Composition according to one Claims 6 to 9, which is in the form of an ointment, a cream, a pomade, a gel, a spray, a lotion, an emulsion or a vesicular dispersion.

11. Use of at least one compound of Formula (I) according to any one of Claims 1 to 4, as an antioxidant.

12. Use of at least one compound of Formula (I) according to any one of Claims 1 to 4, in a cosmetic composition for treating oxidative stress and/or for treating the effects of exposure to sunlight and/or for preventing ageing in particular of the skin or the hair.

13. Use of at least one compound of Formula (I) according to any one of Claims 1 to 4, for the preparation of a pharmaceutical composition
- which is intended to treat oxidative stress associated in particular with certain pathological states, and/or
- which is intended to treat pathological conditions such as cancers, inflammatory conditions, reinfusion ischaemia, iron overloads and degenerative diseases of the nervous system, and/or
- which is intended to treat the effects of exposure to ionizing radiation or solar radiation, and/or.
- which is intended to treat the effects of the use of certain free-radical generating medicinal products, and/or
- which is intended to prevent ageing in particular of the skin or the hair.
